# EUROPEAN PATENT APPLICATION

(11) **EP 3 750 986 A1**
(43) Date of publication of application: **16.12.2020**
(21) Application number: 19751917.6
(22) Date of filing: 04.02.2019
(51) Int. Cl.: C12N 5/074, C12M 3/00

(54) **METHOD AND KIT FOR CULTURING HAIR FOLLICLE'S EPITHELIAL STEM CELLS**

(30) Priority: 07.02.2018 JP 2018020376
(71) Applicant: National University Corporation YOKOHAMA National University, Yokohama-shi Kanagawa 240-8501 (JP); Kanagawa Institute of Industrial Science and Technology, Ebina-shi Kanagawa 243-0435 (JP)
(72) Inventor: FUKUDA, Junji, Yokohama-shi, Kanagawa 240-8501 (JP); KAGEYAMA, Tatsuto, Yokohama-shi, Kanagawa 240-8501 (JP); HIRANO, Sugi, Yokohama-shi, Kanagawa 240-8501 (JP)
(74) Representative: Winter, Brandl, Fürniss, Hübner, Röss, Kaiser, Polte - Partnerschaft mbB
(86) International application number: PCT/JP2019/003903
(87) International publication number: WO 2019/156032

(57) **Abstract**

Provided are a method and kit for growth of hair follicle epithelial stem cells on a large scale while maintaining their hair regeneration ability. The culture method for hair follicle epithelial stem cells includes: an accumulating step of forming an accumulation of hair follicle epithelial stem cells by inoculating a cell culture vessel with the hair follicle epithelial stem cells; a mixing step of producing a mixture of an extracellular matrix component and the accumulation of the hair follicle epithelial stem cells by adding the extracellular matrix component to the accumulation of the hair follicle epithelial stem cells; and a culturing step of culturing the hair follicle epithelial stem cells by adding a medium to the mixture. The culture kit for hair follicle epithelial stem cells includes: a cell culture vessel formed of a material having oxygen permeability; an extracellular matrix component; and a medium.

## Description

### Technical Field

The present invention relates to a culture method and culture kit for hair follicle epithelial stem cells.

### Background Art

Hair follicle epithelial stem cells are stem cells for forming hair. It is known that the hair follicle epithelial stem cells firmly adhere to and spread on a general culture substrate, and when a strong growth switch is turned on, are differentiated into cells that do not have a hair regeneration ability. Hitherto, investigations have been made to devise ways to maintain the hair regeneration ability of the hair follicle epithelial stem cells by a method of culturing hair follicle epithelial stem cells in a culture solution supplemented with a growth factor and various inhibitors (see, for example, Patent Literature 1), or a method of culturing hair follicle epithelial stem cells embedded in Matrigel (see, for example, Non Patent Literature 1).

Meanwhile, the inventors of the present invention have heretofore developed a method for manufacturing a regenerated hair follicle primordium aggregation (see, for example, Patent Literature 2). Specifically, the method includes a step of forming hair follicle primordia by inoculating a microwell plate, which includes regularly arranged microwell portions, with mesenchymal cells and epithelial cells, and co-culturing the mesenchymal cells and the epithelial cells while supplying oxygen thereto.

### Citation List

### Patent Literature

[PTL 1] JP 2012-249556 A
[PTL 2] WO 2017/073625 A1

### Non Patent Literature

[NPL 1] Chacon-Martinez C A et al., "Hair follicle stem cell cultures reveal self-organizing plasticity of stem cells and their progeny.", EMBO, Vol. 36, No. 2, p151-164, 2017.

### Summary of Invention

### Technical Problem

However, there has been a demand for a better culture method capable of growing hair follicle epithelial stem cells on a large scale while maintaining their hair regeneration ability.

The present invention has been made in view of the above-mentioned circumstances, and provides a culture method and culture kit for hair follicle epithelial stem cells, capable of growing hair follicle epithelial stem cells on a large scale while maintaining their hair regeneration ability.

### Solution to Problem

That is, the present invention includes the following aspects.

A culture method for hair follicle epithelial stem cells according to a first aspect of the present invention is a method including: an accumulating step of forming an accumulation of hair follicle epithelial stem cells by inoculating a cell culture vessel with the hair follicle epithelial stem cells; a mixing step of producing a mixture of an extracellular matrix component and the accumulation of the hair follicle epithelial stem cells by adding the extracellular matrix component to the accumulation of the hair follicle epithelial stem cells; and a culturing step of culturing the hair follicle epithelial stem cells by adding a medium to the mixture.

In the culture method for hair follicle epithelial stem cells according to the first aspect, the extracellular matrix component may be type I collagen.

In the culture method for hair follicle epithelial stem cells according to the first aspect, the cell culture vessel may be formed of a material having oxygen permeability.

In the culture method for hair follicle epithelial stem cells according to the first aspect, the material having oxygen permeability may be polydimethylsiloxane.

A culture kit for hair follicle epithelial stem cells according to a second aspect of the present invention is a kit including: a cell culture vessel formed of a material having oxygen permeability; an extracellular matrix component; and a medium.

### Advantageous Effects of Invention

According to the culture method and culture kit for hair follicle epithelial stem cells according to the above-mentioned aspects, hair follicle epithelial stem cells are grown on a large scale while maintaining their hair regeneration ability.

### Brief Description of Drawings

FIG. 1 is a schematic process diagram for illustrating a method of preparing hair follicle epithelial stem cells in Example 1.
FIG. 2 includes a graph and table showing the presence ratio of hair follicle epithelial stem cells in epidermal cells collected from adult mice in Example 1.
FIG. 3A is a graph showing the results of analysis of cells F (CD34-positive cells sorted out by a magnetic cell sorting (MACS) method) by a fluorescence activated cell sorting (FACS) method in Example 1.
FIG. 3B includes a phase contrast micrograph and fluorescence micrographs (upper: nuclear staining image, lower: CD34 staining image) of immunostained cells F (CD34-positive cells sorted out by the magnetic cell sorting (MACS) method) in Example 1. Each scale bar represents 250 µm.
FIG. 4 is a schematic process diagram for comparing culture methods for hair follicle epithelial stem cells in Example 1 and Comparative Example 1.
FIG. 5 includes micrographs of hair follicle epithelial stem cells from the 1st day to the 14th day of culture in Example 1. Each scale bar represents 1 mm.
FIG. 6 is a graph showing the expression amounts of CD34 gene in hair follicle epithelial stem cells on the 14th day of culture in Example 1 and Comparative Example 1.
FIG. 7A includes micrographs of hair follicle epithelial stem cells from the 1st day to the 14th day of culture cultured by a two-dimensional culture method in Comparative Example 1. Each scale bar represents 1 mm.
FIG. 7B includes micrographs of hair follicle epithelial stem cells from the 1st day to the 14th day of culture cultured by a related-art Matrigel-embedded culture method in Comparative Example 1. Each scale bar represents 1 mm.
FIG. 8 is a graph showing the expression amounts of CD34 gene in hair follicle epithelial stem cells before culture and on the 14th day of culture (two-dimensional culture and Matrigel-embedded culture) in Comparative Example 1.
FIG. 9 is a schematic process diagram for illustrating a production method for an oxygen-permeable cell culture vessel (polydimethylsiloxane (PDMS) spheroid chip) in Example 2.
FIG. 10 includes micrographs of hair follicle epithelial stem cells from the 1st day to the 14th day of culture in Example 2. Each scale bar represents 1 mm.
FIG. 11 is a graph showing the expression amounts of CD34 gene in hair follicle epithelial stem cells on the 14th day of culture in Examples 1 and 2 and Comparative Examples 1 and 2.
FIG. 12 is a schematic configuration diagram for comparing cell culture vessels (culture conditions) in Comparative Example 1 and Comparative Example 2.
FIG. 13 includes micrographs of hair follicle epithelial stem cells from the 1st day to the 14th day of culture in Comparative Example 2. Each scale bar represents 1 mm.

### Description of Embodiments

### <Culture Method for Hair Follicle Epithelial Stem Cells>

A culture method for hair follicle epithelial stem cells according to one embodiment of the present invention is a method including an accumulating step, a mixing step, and a culturing step.

The accumulating step includes inoculating a cell culture vessel with hair follicle epithelial stem cells to form an accumulation thereof.

The mixing step includes adding an extracellular matrix component to the accumulation of the hair follicle epithelial stem cells to produce a mixture of the extracellular matrix component and the accumulation of the hair follicle epithelial stem cells.

The culturing step includes adding a medium to the mixture, and culturing the hair follicle epithelial stem cells.

According to the culture method according to this embodiment, as described later in Examples, hair follicle epithelial stem cells are grown on a large scale while maintaining their hair regeneration ability.

In a related-art culture method for hair follicle epithelial stem cells, hair follicle epithelial stem cells are mixed with an extracellular matrix component, such as Matrigel, at the time of inoculating the cell, and the hair follicle epithelial stem cells are cultured in a state of being dispersed.

Meanwhile, in a living body, hair follicle epithelial stem cells are present in an environment where the cells are densely populated, and extracellular matrix components are present between the cells. Therefore, in the culture method according to this embodiment, an environment similar to that in the living body is reproduced by forming a cell accumulation in the accumulating step and mixing the cell accumulation with the extracellular matrix component in the subsequent mixing step. Consequently, as described later in Examples, hair follicle epithelial stem cells having a more excellent hair regeneration ability than those obtained in the related-art culture method are obtained.

As used herein, the term "cell accumulation" means a mass of the cells, which have been inoculated into the cell culture vessel, piled up on the bottom surface of the culture vessel by gravity or the like.

Details about each step of the culture method according to this embodiment will be described below.

### [Accumulating Step]

First, hair follicle epithelial stem cells are inoculated in a cell culture vessel, and then a static culture of the cells is performed. The cells are deposited by gravity to form an accumulation of the cells. The number of the cells to be inoculated in the cell culture vessel is appropriately adjusted depending on the size of the cell culture vessel.

### (Hair Follicle Epithelial Stem Cells)

The origin of the hair follicle epithelial stem cells to be used in the culture method according to this embodiment is an animal, preferably a vertebrate, more preferably a mammal. Examples of the mammal include, but are not limited to: humans, chimpanzees, and other primates; and domestic animals, pet animals, and experimental animals, such as dogs, cats, rabbits, horses, sheep, goats, cattle, pigs, rats (including nude rats), mice (including nude mice and SCID mice), and guinea pigs. Of those, the origin of the cells is preferably a human.

The hair follicle epithelial stem cells may be isolated from a skin tissue of a subject animal, or may be induced from pluripotent cells. Examples of the pluripotent cells include embryonic stem (ES) cells, embryonic germ (EG) cells, and induced pluripotent stem (iPS) cells.

In addition, the hair follicle epithelial stem cells to be used in the culture method according to this embodiment may be cells of a single type, or may be mixed with cells present around hair follicle epithelial stem cells in a skin tissue (e.g., pigment stem cells and epidermal cells).

The hair follicle epithelial stem cells may be identified on the basis of whether or not a marker protein (e.g., CD34) for hair follicle epithelial stem cells is expressed. Specifically, for example, cells expressing CD34 may be identified using a known method (e.g., fluorescence activated cell sorting (FACS) analysis, magnetic cell sorting (MACS) analysis, and an immunostaining method, each using an anti-CD34 antibody).

With regard to culture conditions in the accumulating step, a culture time may be 10 minutes or more and 24 hours or less (preferably 1 hour or more and 2 hours or less), and a culture temperature may be 25°C or more and less than 40°C (preferably 37°C) . In addition, the culture may be performed under the condition of, for example, about 5% CO₂.

### (Cell Culture Vessel)

The cell culture vessel is preferably a substrate in which a plurality of wells are regularly arranged, from the viewpoints of ease of observation and screening efficiency. In the present application, each of the wells arranged in the substrate is regarded as the cell culture vessel. A commercially available cell culture vessel may be used as such a cell culture vessel, or such a cell culture vessel may be produced by, for example, a method described in Patent Literature 3 (WO 2017/073625 A1).

In addition, the density of the wells in the substrate may be, for example, 20 wells/cm² or more and 500 wells/cm² or less, 50 wells/cm² or more and 250 wells/cm² or less, or 100 wells/cm² or more and 200 wells/cm² or less. When the density falls within the above-mentioned ranges, culture can be performed under a state in which the hair follicle epithelial stem cells are arranged at a density similar to the density of pores of a mammal (in particular, pores of a primate including a human).

The diameter and depth of the opening portion of each of the wells are not particularly limited as long as the size thereof allows for the accommodation and culture of the accumulation of the hair follicle epithelial stem cells. The diameter may be similar to the size of a pore of a mammal, and may be, for example, 20 µm or more and 1 mm or less. In addition, the depth may be, for example, 1 mm or less.

A material for the cell culture vessel may be a material suitable for cell culture, and is not particularly limited. Examples thereof include transparent glass and polymer materials. Of those, a polymer material having oxygen permeability is preferred. Specific examples of the polymer material having oxygen permeability include a fluorine resin and a silicon rubber (e.g., poly(dimethylsiloxane): PDMS). Those materials may be used alone or in combination thereof.

As used herein, the term "oxygen permeability" refers to a property of allowing molecular oxygen to permeate the material and reach the interior of each of the wells of the cell culture vessel. A specific oxygen permeation rate may be about 100 cm³/m²·24 h·atm or more and about 5,000 cm³/m²·24 h·atm or less, about 1,100 cm³/m²·24 h·atm or more and about 3,000 cm³/m²·24 h·atm or less, or about 1,250 cm³/m²·24 h·atm or more and about 2,750 cm³/m²·24 h·atm or less. "24 h" means 24 hours, and "atm" means a unit of atmospheric pressure. That is, the unit "cm³/m²·24 h·atm" represents the volume (cm³) of oxygen permeating the material in 24 hours per 1 m² under an environment of 1 atm. When a cell culture vessel formed of a material having an oxygen permeation rate falling within the above-mentioned ranges is used, a sufficient amount of oxygen can be supplied to the hair follicle epithelial stem cells, and hence hair follicle epithelial stem cells having a more excellent hair regeneration ability are obtained.

In addition, as described later in Examples, a commercially available cell culture vessel may be used as the cell culture vessel, or the cell culture vessel may be produced from scratch by producing a mold and using PDMS as a raw material. An example of the commercially available cell culture vessel is a PrimeSurface (trademark) 96U plate manufactured by Sumitomo Bakelite Co., Ltd.

### (Medium)

The medium is not particularly limited, and may be a basal medium containing components required for the survival and growth of cells (an inorganic salt, a carbohydrate, a hormone, an essential amino acid, a non-essential amino acid, and a vitamin) and the like.

The inorganic salt to be contained in the medium serves to help maintain the osmotic pressure equilibrium of cells and to help regulate the membrane potential thereof.

The inorganic salt is not particularly limited, and examples thereof include salts of, for example, calcium, copper, iron, magnesium, potassium, sodium, and zinc. The salt is typically used in the form of any of a chloride, a phosphate, a sulfate, a nitrate, and a bicarbonate.

In general, the osmolality of the inorganic salt in the medium may be, for example, 200 mOsm/kg or more and 400 mOsm/kg or less, 280 mOsm/kg or more and 350 mOsm/kg or less, 280 mOsm/kg or more and 310 mOsm/kg or less, 280 mOsm/kg or more and less than 300 mOsm/kg, or 280 mOsm/kg.

The carbohydrate is not particularly limited, and examples thereof include glucose, galactose, maltose, and fructose.

In general, the concentration of the carbohydrate (preferably D-glucose) in the medium is preferably 0.5 g/L or more and 2 g/L or less.

The amino acid is not particularly limited, and examples thereof include L-alanine, L-arginine, L-asparagine, L-aspartic acid, L-cysteine, L-cystine, L-glutamic acid, L-glutamine, L-glycine, L-histidine, L-isoleucine, L-leucine, L-lysine, L-methionine, L-phenylalanine, L-proline, L-serine, L-threonine, L-tryptophan, L-tyrosine, L-valine, and combinations thereof.

In general, the concentration of glutamine in the medium may be 0.05 g/L or more and 1 g/L or less (typically 0.1 g/L or more and 0.75 g/L or less). The concentration of the amino acid other than glutamine in the medium may be 0.001 g/L or more and 1 g/L or less (typically 0.01 g/L or more and 0.15 g/L or less). The amino acid may be derived from synthesis.

The vitamin is not particularly limited, and examples thereof include thiamine (vitamin B1), riboflavin (vitamin B2), niacinamide (vitamin B3), D-pantothenic acid hemicalcium (vitamin B5), pyridoxal/pyridoxamine/pyridoxine (vitamin B6), folic acid (vitamin B9), cyanocobalamin (vitamin B12), ascorbic acid (vitamin C), calciferol (vitamin D2), DL-α-tocopherol (vitamin E), biotin (vitamin H), menadione (vitamin K), choline chloride, and myo-inositol.

The medium may further contain an antibiotic, a serum, a growth factor, a hormone, or a ROCK inhibitor.

Examples of the antibiotic include antibiotics used for culture of typical animal cells, such as gentamicin, amphotericin, ampicillin, minomycin, kanamycin, penicillin, streptomycin, gentacin, tylosin, and aureomycin. Those antibiotics may be contained alone or in combination thereof.

In general, the concentration of the antibiotic in the medium is not particularly limited, and may be, for example, 0.1 µg/mL or more and 100 µg/mL or less.

Examples of the serum include, but are not limited to, fetal bovine/calf serum (FBS/FCS), newborn calf serum (NCS), calf serum (CS), and horse serum (HS).

In general, the concentration of the serum in the medium may be, for example, 2 mass% or more and 10 mass% or less.

Examples of the growth factor include, but are not limited to, a cell growth factor and a cell adhesion factor.

More specific examples of the growth factor include an epidermal growth factor (EGF), an acidic fibroblast growth factor (aFGF), a basic fibroblast growth factor (bFGF), an insulin-like growth factor-1 (IGF-1), a macrophage-derived growth factor (MDGF), a platelet-derived growth factor (PDGF), a tumor angiogenesis factor (TAF), and a vascular endothelial growth factor (VEGF). Those growth factors may be contained alone or in combination thereof.

In general, the concentration of the growth factor in the medium is not particularly limited, and may be, for example, 1 ng/mL or more and 10 µg/mL or less.

Examples of the hormone include insulin, glucagon, triiodothyronine, and an adrenocortical hormone (e.g., hydrocortisone). Those hormones may be contained alone or in combination thereof.

In general, the concentration of the hormone in the medium is not particularly limited, and may be, for example, 1 ng/mL or more and 10 µg/mL or less.

In addition, bovine pituitary extract (BPE) may be used as a medium additive containing growth factors and hormones.

Examples of the ROCK inhibitor include HA-1077, Y-27632, Thiazovivin, GSK429286A, RKI-1447, GSK180736A, HA-1100, Y-39983, AR-13324, GSK269962, AT13148, K-115, KD025, ZINC00881524, and salts thereof.

Specifically, a known basal medium for epithelial cells containing calcium chloride, free of serum, and supplemented with an epidermal growth factor, and as required, any antibiotic and any hormone may be used as the medium.

Examples of the known basal medium for epithelial cells include HuMedia-KB2 (manufactured by Kurabo Industries Ltd.), Keratinocyte Basal Medium 2 (manufactured by PromoCell GmbH), and EpiLife (trademark) Medium (manufactured by Thermo Fisher Scientific).

In addition, examples of the epithelial cell growth medium containing an epidermal growth factor, any antibiotic, and any hormone include HuMedia-KG2 (manufactured by Kurabo Industries Ltd.) and Keratinocyte Growth Medium 2 (manufactured by PromoCell GmbH). In addition, any such epithelial cell growth medium containing an epidermal growth factor, any antibiotic, and any hormone may be further supplemented with, for example, any growth factor and any ROCK inhibitor.

### [Mixing Step]

Then, an extracellular matrix component is added to the accumulation of the hair follicle epithelial stem cells and a mixture of the extracellular matrix component and the accumulation of the hair follicle epithelial stem cells is produced. Thus, in the subsequent culturing step, the hair follicle epithelial stem cells are cultured in an environment similar to that in a living body. In the mixing step, the extracellular matrix component may be gelated, or may not be gelated.

The concentration of the extracellular matrix component in the solution may be appropriately adjusted depending on the presence or absence of the gelation and the hardness of gel. In addition, when the extracellular matrix component is gelated, a gelation time may also be appropriately adjusted depending on the required hardness of the gel. Various conditions, such as a gelation temperature, are not particularly limited, and there is given, for example, a method involving performing culture in a CO₂ incubator at 37°C. In addition, examples of a cell culture vessel to be used in the mixing step include the same ones as those exemplified in the foregoing section "[Accumulating Step]".

### (Extracellular Matrix Component)

Examples of the extracellular matrix component include collagen (e.g., type I, type II, type III, type IV, type V, type XI, and type XVII), a basal membrane component (product name: Matrigel) reconstructed from a mouse EHS tumor extract (including type IV collagen, laminin, heparan sulfate proteoglycan, and the like), fibrin, glycosaminoglycan, hyaluronic acid, and proteoglycan. As other polymers derived from natural substances, gelatin, agar, agarose, and the like may also be used. A hydrogel may be produced by selecting, for example, a component, such as a salt, the concentration thereof, and a pH, that are optimal for the gelation of the above-mentioned materials. In addition, those polymers derived from natural substances may be used alone or in combination thereof.

Of those, the extracellular matrix component is preferably collagen (in particular, type I collagen). The extracellular matrix component containing collagen results in a composition closer to that of the skin, and hence high hair follicle regeneration efficiency are achieved.

The extracellular matrix component may be suspended in a solvent. Examples of the solvent in which the extracellular matrix component is suspended include a serum-free medium, such as Ham's Nutrient Mixtures F-10 or Ham's Nutrient Mixtures F-12, and a buffer solution for reconstructing the extracellular matrix component (e.g., a buffer solution formed of sodium hydroxide, sodium hydrogen carbonate, and HEPES-Buffer).

### [Culturing step]

Then, a medium is added to the mixture produced in the mixing step, and the mixture is cultured. Examples of a cell culture vessel and the medium to be used in the culturing step include the same ones as those exemplified in the foregoing section "[Accumulating Step]". With regard to culture conditions, a culture time may be 3 days or more and 21 days or less (preferably 10 days or more and 14 days or less), and a culture temperature may be 25°C or more and less than 40°C (preferably 37°C). In addition, the culture may be performed under the condition of, for example, about 5% CO₂.

In the culturing step, as described later in Examples, as the number of days of culture increases, the hair follicle epithelial stem cells adhere to and aggregate with each other to form one aggregate.

Through the culturing step, hair follicle epithelial stem cells having excellent hair regeneration ability are obtained on a large scale.

### [Other Steps]

In addition, the culture method according to this embodiment may further include, after the culturing step, for example, a confirming step of confirming the expression of a marker protein in hair follicle epithelial stem cells.

Examples of a method of confirming the expression of the marker protein in hair follicle epithelial stem cells include the same methods as those exemplified in the foregoing section "(Hair Follicle Epithelial Stem Cells)".

In addition, the culture method according to this embodiment may further include, after the culturing step, for example, an isolating step of isolating the obtained hair follicle epithelial stem cells into single cells. As a method for the isolation, there is given, for example, a method involving performing enzymatic treatment with trypsin or the like.

### <Culture Kit for Hair Follicle Epithelial Stem Cells>

A culture kit for hair follicle epithelial stem cells according to one embodiment of the present invention includes a cell culture vessel formed of a material having oxygen permeability, an extracellular matrix component, and a medium.

According to the culture kit according to this embodiment, hair follicle epithelial stem cells are grown on a large scale while maintaining their hair regeneration ability.

Examples of the cell culture vessel formed of a material having oxygen permeability, the extracellular matrix component, and the medium, which are included in the culture kit according to this embodiment, include the same ones as those exemplified in the foregoing section "<Culture Method for Hair Follicle Epithelial Stem Cells>".

### [Other Components]

The culture kit according to this embodiment may further include, for example, an antibody against a marker protein in hair follicle epithelial stem cells (e.g., anti-CD34 antibody), in addition to the cell culture vessel formed of a material having oxygen permeability, the extracellular matrix component, and the medium. Consequently, it is confirmed whether or not the cells obtained by culture are hair follicle epithelial stem cells (whether or not the cells maintain a hair regeneration ability).

In addition, the culture kit according to this embodiment may further include an enzyme, such as trypsin, in addition to the cell culture vessel formed of a material having oxygen permeability, the extracellular matrix component, and the medium. Consequently, hair follicle epithelial stem cells obtained using the culture kit according to this embodiment are isolated into single cells.

### <Use Applications of Hair Follicle Epithelial Stem Cells>

Hair follicle epithelial stem cells obtained using the culture method and the culture kit according to the embodiments of the present invention may, for example, construct hair follicle primordia by being co-cultured with mesenchymal cells, such as hair papilla cells. Hair can be regenerated by transplanting the hair follicle primordia.

The hair follicle epithelial stem cells obtained using the culture method and the culture kit according to the embodiments of the present invention may, for example, regenerate skin by being transplanted to a wound site of a subject animal.

### Examples

Now, the present invention will be described by way of Examples, but the present invention is not limited to the following Examples.

### [Example 1] Culture of Hair Follicle Epithelial Stem Cells 1

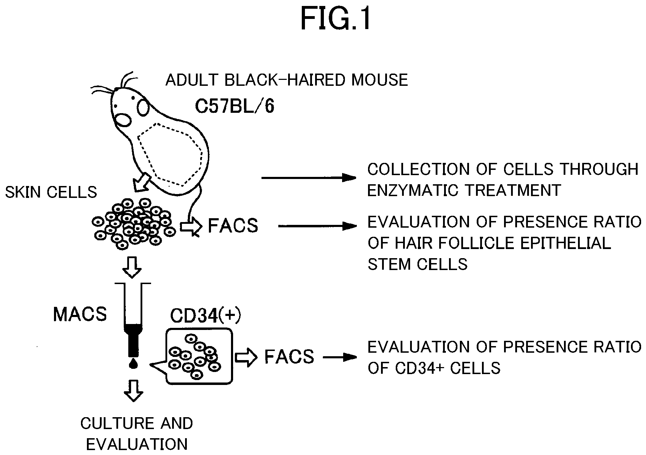

First, skin cells were collected from adult mice and evaluated for the presence ratio of hair follicle epithelial stem cells, and then the hair follicle epithelial stem cells are isolated and used in subsequent tests (see FIG. 1).

### 1. Preparation of Hair Follicle Epithelial Stem Cells (1) Collection of Mouse Epithelial Cells

Dorsal skin was collected from 6- to 8-week-old male adult mice (C57BL/6jjcl, purchased from Charles River), and was washed with Dulbecco's phosphate-buffered saline (DPBS) (manufactured by Gibco) containing 100 mg/L penicillin-streptomycin (P/S) (manufactured by Gibco). Then, an adipose tissue was surgically removed with a scalpel to provide a skin tissue. The skin tissue was finely cut into 1 cm to 1.5 cm squares, and incubated with 0.25% trypsin (manufactured by Gibco) at 37°C for 70 minutes with a dermis side directed downward. Then, a dermal layer was surgically removed using tweezers. The skin tissue was loaded into a 50 mL centrifuge tube together with the solution in which the enzymatic treatment had been performed, and pipetting was performed to separate the cells from each other. Then, the whole solution containing the cells was passed through a 70 µm cell strainer (manufactured by BD Falcon), and further passed through a 40 µm cell strainer (manufactured by BD Falcon). Then, the cells were collected by centrifugation at 1,000 rpm for 3 minutes. The collected cells were suspended in Humedia-KG2 medium (manufactured by Kurabo Industries Ltd.).

### (2) Confirmation of Presence Ratio of CD34-positive Cells

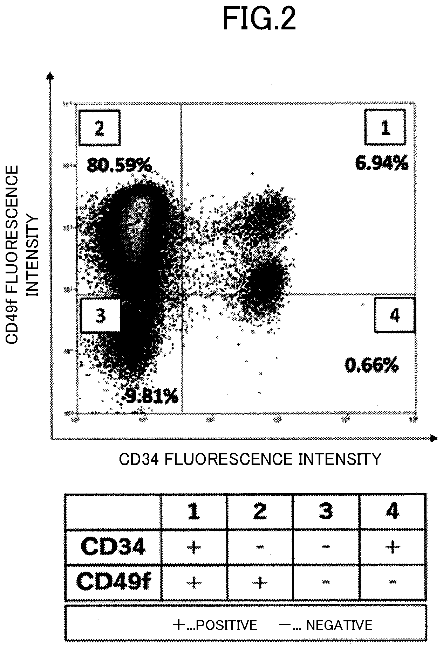

The cell suspension prepared in (1) was centrifuged at 1,000 rpm for 3 minutes, and the supernatant was removed. Then, an operation involving suspending the cells in 5 mL of PBS, centrifuging the suspension at 1,000 rpm for 3 minutes, and removing the supernatant was performed twice to completely remove the medium. PBS was added to the resultant, and the suspension was dispensed into a 1.5 mL tube at 1×10⁶ cells/500 mL. Then, an FITC-labeled anti-CD34 antibody (manufactured by BD) and a PE-labeled anti-CD49f antibody (manufactured by R&D Systems) were added as antibodies for fluorescence activated cell sorting (FACS), and the cells were stained on ice in a dark place for 30 minutes. After the staining, the resultant was centrifuged at 1,000 rpm for 3 minutes, and the supernatant was removed. Then, an operation involving suspending the cells in 5 mL of PBS, centrifuging the suspension at 1,000 rpm for 3 minutes, and removing the supernatant was performed twice to completely remove the solution containing the antibodies for FACS. For nuclear staining of dead cells, 7-amino-actinomycin D (7-AAD) was added. Then, the resultant was passed into a test tube with a 30 µm filter, followed by analysis using an automated cell analysis and sorting apparatus (FACS apparatus) (manufactured by Beckman Coulter) . The results are shown in FIG. 2.

It was found from FIG. 2 that cells expressing both of CD34 and CD49f were present at 6.94% in the group of epidermal cells collected from the mouse skin. In addition, in a previous investigation (see Non Patent Literature 1 described above), there is a report that the presence ratio of cells expressing both of CD34 and CD49f in the group of epidermal cells of mice of the strain used is from about 5% to about 10%. The present result fell within the reported value range.

In addition, the presence ratio of CD34(+)/CD49f(-) cells corresponding to region "4" in FIG. 2 was 0.66%, which was judged to be negligibly small. Therefore, CD34-positive (+) cells (cells classified as falling within regions "1" and "4" in FIG. 2) were used in the following experiments.

### (3) Isolation of CD34-positive Cells

Then, the CD34-positive (+) cells were collected by the following procedure. All operations were performed on ice.
(3-1) A cell suspension containing the collected mouse epithelial cells at from 1.0 to 1.5×10⁷ cells was centrifuged at 1,000 rpm for 3 minutes, and then the supernatant was removed. Then, 300 µL of magnetic cell sorting (MACS)/FACS buffer (manufactured by Miltenyi Biotec) was added.
(3-2) Three 5 mL tubes were prepared, and the cell suspension prepared in (3-1) was dispensed at from 10 µL to 15 µL per tube. The tubes were defined as a tube 1, a tube 2, and a tube 3, respectively, and subsequently, operations described in "(4) Procedure for Each Tube)" were performed.
(3-3) Then, after the dispensation in (3-2), 3 mL of MACS/FACS buffer was added to the remaining cell suspension, the whole was centrifuged at 1,000 rpm for 3 minutes, and the supernatant was removed. Then, 300 µL of MACS/FACS buffer was added. After that, 30 µL of an anti-mouse CD34 antibody (manufactured by eBioscience) was added, and the whole was left to rest in a dark place at 4°C for 30 minutes.
(3-4) 5 mL of MACS/FACS buffer was added, and then the whole was centrifuged at 1,000 rpm for 3 minutes. The supernatant was removed, and the cells were resuspended in 300 µL of MACS/FACS buffer. After that, 30 µL of an FITC-labeled anti-rat IgG antibody (manufactured by Jackson Immuno Research) was added, and the whole was left to rest in a dark place at 4°C for 30 minutes.
(3-5) An operation involving adding 5 mL of MACS/FACS buffer, then centrifuging the whole at 1,000 rpm for 3 minutes, and removing the supernatant was performed twice to completely remove the FITC-labeled anti-rat antibody. Then, the cells were resuspended in 300 µL of MACS/FACS buffer, then 30 µL of anti-FITC antibody-bound microbeads (manufactured by Miltenyl Biotec) were added, and the whole was left to rest in a dark place at 4°C for 30 minutes.
(3-6) 5 mL of MACS/FACS buffer was added, and then the whole was centrifuged at 1,000 rpm for 3 minutes. The supernatant was removed, and the cells were resuspended in 500 µL of MACS/FACS buffer, followed by storage at 4°C. Before the sample was made to flow with a magnetic cell sorting apparatus (MACS apparatus) (manufactured by Miltenyl Biotec), 15 µL of the sample was transferred to a 15 mL tube and diluted to 600 µL by adding MACS/FACS buffer (the tube containing this sample is hereinafter sometimes referred to as "tube 4"). Subsequently, an operation described in "(4) Procedure for Each Tube)" was performed.
(3-7) The sample prepared in (3-6) was separated into labeled cells and unlabeled cells in accordance with the protocol of Miltenyi Biotec.
(3-8) 600 µL of the unlabeled cell suspension was collected in a 15 mL tube and stored at 4°C (the tube containing the unlabeled cell suspension is hereinafter sometimes referred to as "tube 5"). Subsequently, an operation described in "(4) Procedure for Each Tube)" was performed.
(3-9) 400 µL of the labeled cell suspension was collected in a 15 mL tube and stored at 4°C (the tube containing the labeled cell suspension is hereinafter sometimes referred to as "tube 6"). Subsequently, an operation described in "(4) Procedure for Each Tube)" was performed.

### (4) Procedure for Each Tube (Preparation of Sample for FACS)

### (4-1) Tube 1 (Preparation of Unstained Control)

600 µL of MACS/FACS buffer was added to the tube 1, followed by storage at 4°C.

### (4-2) Tube 2 (Preparation of Background Control)

(4-2-1) MACS/FACS buffer was added so that the volume of the cell suspension in the tube 2 became 50 µL. Further, 5 µL of an FITC-labeled anti-rat IgG antibody was added, and the whole was left to rest in a dark place at 4°C for 30 minutes.
(4-2-2) 5 mL of MACS/FACS buffer was added, and then the whole was centrifuged at 1,000 rpm for 3 minutes. The supernatant was removed, and the cells were resuspended in 50 µL of MACS/FACS buffer. After that, 5 µL of anti-FITC antibody-bound microbeads were added, and the whole was left to rest in a dark place at 4°C for 30 minutes.
(4-2-3) 5 mL of MACS/FACS buffer was added, and then the whole was centrifuged at 1,000 rpm for 3 minutes. The supernatant was removed, and 600 µL of MACS/FACS buffer was added, followed by storage at 4°C.

### (4-3) Tube 3 (Preparation of Isotype Control)

(4-3-1) MACS/FACS buffer was added so that the volume of the cell suspension in the tube 3 became 50 µL. Further, 5 µL of a rat IgG isotype antibody (PE-labeled rat IgG antibody) (manufactured by R&D Systems) was added, and the whole was left to rest in a dark place at 4°C for 30 minutes.
(4-3-2) 5 mL of MACS/FACS buffer was added, and then the whole was centrifuged at 1,000 rpm for 3 minutes. The supernatant was removed, and the cells were resuspended in 50 µL of MACS/FACS buffer. After that, 5 µL of an FITC-labeled anti-rat IgG antibody was added, and the whole was left to rest in a dark place at 4°C for 30 minutes.
(4-3-3) 5 mL of MACS/FACS buffer was added, and then the whole was centrifuged at 1,000 rpm for 3 minutes. The supernatant was removed, and the cells were resuspended in 50 µL of MACS/FACS buffer. After that, 5 µL of anti-FITC antibody-bound microbeads were added, and the whole was left to rest in a dark place at 4°C for 30 minutes.
(4-3-4) 5 mL of MACS/FACS buffer was added, and the whole was centrifuged at 1,000 rpm for 3 minutes. The supernatant was removed, and 600 µL of MACS/FACS buffer was added, followed by storage at 4°C.

### (4-4) Tube 4 (Preparation of Presort Sample)

600 µL of MACS/FACS buffer was added to the tube 4, followed by storage at 4°C.

### (4-5) Tube 5 (Preparation of Unlabeled Cells)

400 µL of MACS/FACS buffer was added to the tube 5, followed by storage at 4°C.

### (4-6) Tube 6 (Preparation of Labeled Cells)

600 µL of MACS/FACS buffer was added to the tube 6, followed by storage at 4°C.

### (5) FACS Analysis

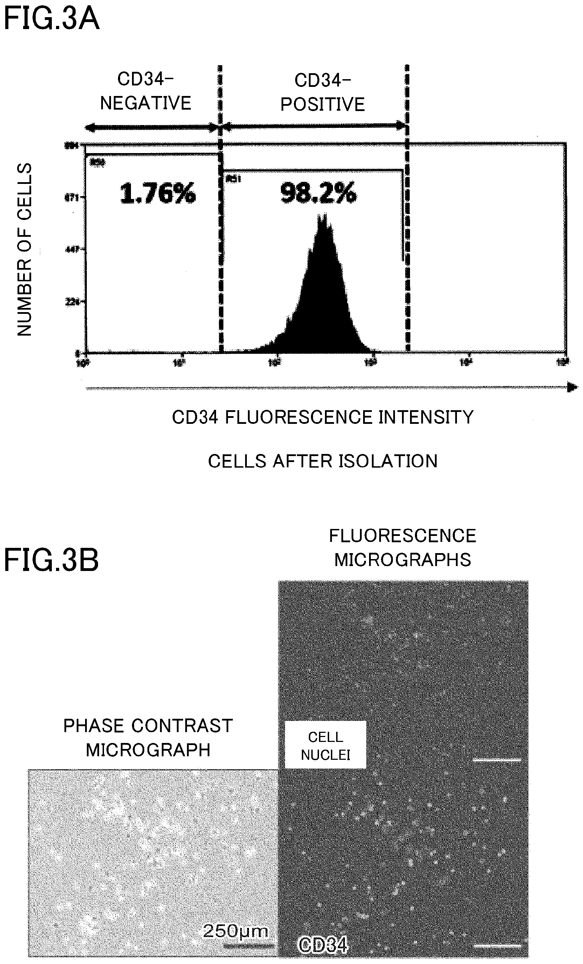

Then, the samples for FACS analysis in the tubes 1 to 6 prepared in (4) were each subjected to analysis using a FACS apparatus to count the number of cells at each fluorescence intensity. The results are shown in Table 1 below and FIG. 3A (graph of the FACS analysis results of the labeled cells).

### (6) Immunofluorescent Staining

In addition, the cell suspension in the tube 6 was subjected to immunofluorescent staining of CD34 and observed using an inverted phase contrast fluorescence microscope (manufactured by Olympus, IX-71). The results are shown in FIG. 3B.

**Table 1**

| Cells | Tube | Sample name | CD34(+) [%] | CD34 (-) [%] |
|---|---|---|---|---|
| A | 1 | Unstained control | 0.00 | 100.0 |
| B | 2 | Background control | 0.02 | 99.96 |
| C | 3 | Isotype control | 0.09 | 99.91 |
| D | 4 | Presort sample | 6.90 | 93.15 |
| E | 5 | Unlabeled cells | 4.07 | 95.97 |
| F | 6 | Labeled cells | 98.26 | 1.76 |

It was found from Table 1 and FIG. 3A that the purity of the CD34-positive (+) cells was 98.26%.

In addition, as shown in FIG. 3B, the cells adhered and survived on the culture dish, and maintained the expression of CD34 even after cell adhesion.

Thus, it was confirmed that hair follicle epithelial stem cells maintaining the expression of CD34 were able to be isolated from the mouse skin tissue.

### 2. Culture of Hair Follicle Epithelial Stem Cells (1) Preparation of Epithelial Cell Culture Medium

Humedia-KG2 medium (manufactured by Kurabo Industries Ltd.) was supplemented with 5 mL of fetal bovine serum (FBS) (manufactured by Sigma), 16 mg of L-glutamine (manufactured by Wako Pure Chemical Industries, Ltd.), 100 µL of 10 ng/pL recombinant mouse fibroblast growth factor 2 (FGF2) (manufactured by R&D Systems), 10 µL of 100 µg/mL vascular endothelial growth factor-A (VEGF-A) (manufactured by R&D Systems), and 275 µL of 1 mM Y-27632 (manufactured by Wako Pure Chemical Industries, Ltd.), and the whole was thoroughly stirred. Then, the mixture was passed through a sterile filter to prepare Humedia-KG2 medium containing 20 ng/mL FGF2, 20 ng/mL VEGF-A, and 5 µM Y-27632 (hereinafter sometimes referred to as "epithelial cell culture medium").

### (2) Accumulating Step

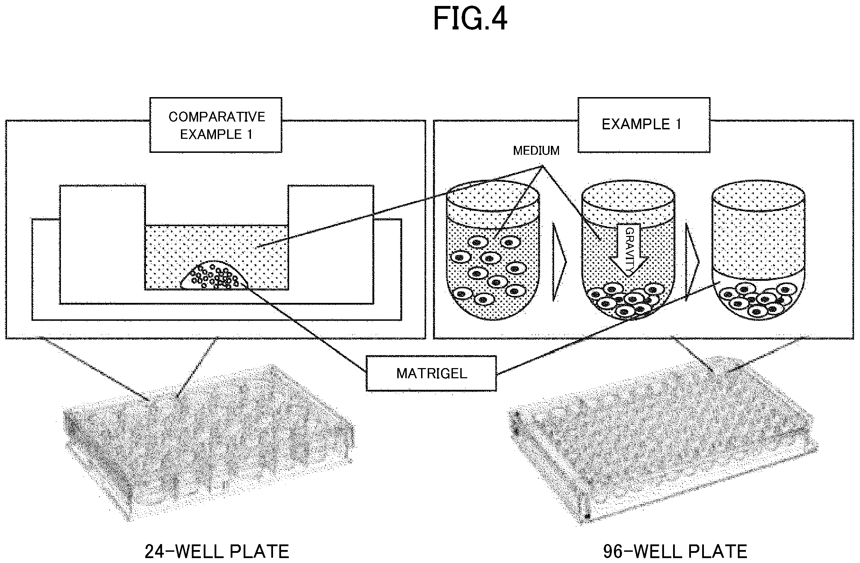

8.0×10⁴ hair follicle epithelial stem cells obtained in "1." were suspended in 100 µL of the epithelial cell culture medium prepared in (1) to prepare a cell suspension. Then, the cell suspension was dispensed in each well of a non-adherent 96U-well plate (manufactured by Sumitomo Bakelite Co., Ltd., radius of curvature: 4.5 mm) and incubated at 37°C for about 30 minutes or more and about 24 hours or less. It was confirmed that the cells were deposited on the bottom surface of the culture vessel by gravity to form an accumulation of the cells (see FIG. 4).

### (3) Mixing Step

Then, only the medium was gently removed, and Matrigel (trademark) (the description "trademark" is hereinafter omitted) (manufactured by Corning) was gently added from above the accumulation of the cells. The gel was hardened by incubation at 37°C for 30 minutes. Thus, a mixture of Matrigel and the accumulation of the hair follicle epithelial stem cells was obtained.

### (4) Culturing step

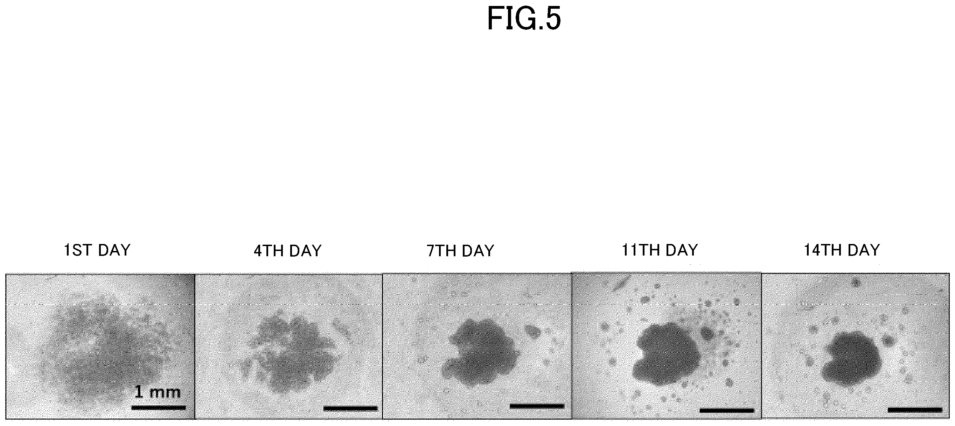

Then, the epithelial cell culture medium was added to the mixture of Matrigel and the accumulation of the hair follicle epithelial stem cells, and the hair follicle epithelial stem cells were cultured for 14 days, to evaluate whether the density of the cells was useful for maintaining the regeneration efficiency of the hair follicle epithelial stem cells. The morphological changes of the cells over time (on the 1st, 4th, 7th, 11th, and 14th days of culture) were observed using an inverted phase contrast fluorescence microscope (manufactured by Olympus, IX-71). The results are shown in FIG. 5.

As shown in FIG. 5, the cells were close together, but the cells were in a state of being separate from each other, on the 1st day of culture. However, the cells started to adhere to and aggregate with each other on the 4th day of culture, and almost all the cells formed one aggregate on the 7th day of culture.

### (5) Analysis of Gene Expression Amount of CD34

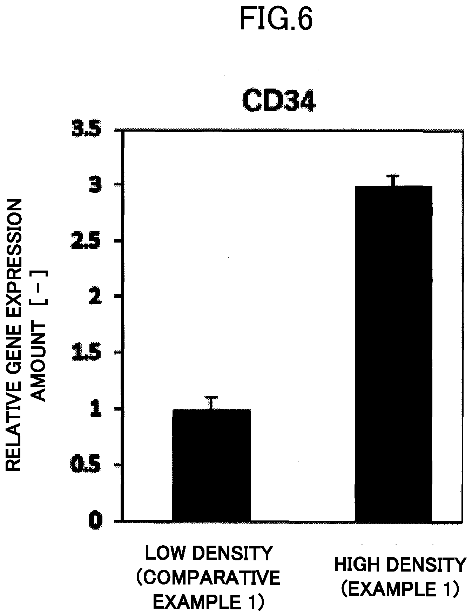

Then, the cells on the 14th day of culture were collected, and the gene expression amount of CD34 was measured by an RT-PCR method through use of a real-time PCR system Step One (trademark) (manufactured by Applied Biosystems) and SYBR (trademark) Green Real-Time PCR Master Mixes (manufactured by Thermo Fisher Scientific). The procedure was performed in accordance with the protocol of SYBR (trademark) Green Real-Time PCR Master Mixes. In addition, primers shown in Table 2 below were used. The results are shown in FIG. 6. FIG. 6 shows the relative gene expression amount of CD34 in Example 1 (high density) when the gene expression amount of CD34 in Comparative Example 1 (low density) to be described below is defined as 1. The discussion of the gene expression amount of CD34 in Example 1 shown in FIG. 6 will be described in Comparative Example 1 below.

**Table 2**

| | | Base sequence (5'→3') | SEQ ID NO: |
|---|---|---|---|
| CD34 | Forward primer | 5'-TgggTCAAgTTgTggTgggAA-3' | 1 |
| | Reverse primer | 5'-gAAgAggCgAgAgAggAgAAATg-3' | 2 |
| GAPDH | Forward primer | 5'-AgAACATCATCCCTgCATCC-3' | 3 |
| | Reverse primer | 5'-TCCACCACCCTgTTgCTgTA-3' | 4 |

### [Comparative Example 1] Culture of Hair Follicle Epithelial Stem Cells 2

### 1. Preparation of Hair Follicle Epithelial Stem Cells

Hair follicle epithelial stem cells were prepared using the same method as in "1." of Example 1.

### 2. Culture of Hair Follicle Epithelial Stem Cells (1) Preparation of Epithelial Cell Culture Medium

An epithelial cell culture medium was prepared using the same method as in "2.(1)" of Example 1.

### (2) Culture of Hair Follicle Epithelial Stem Cells by Related-art Method

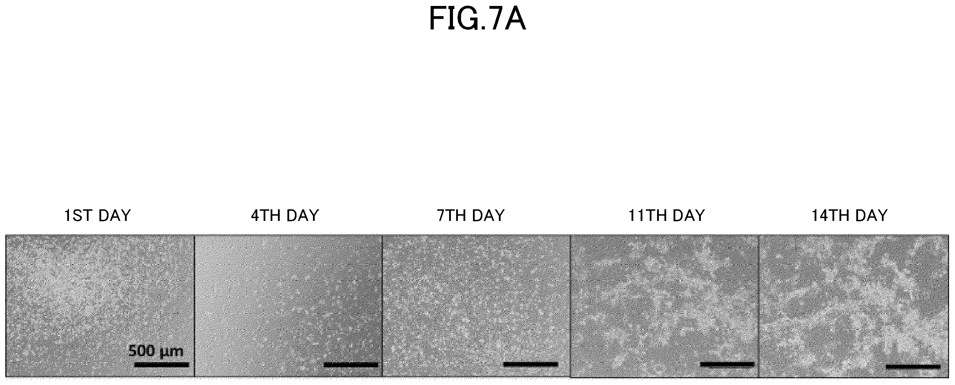

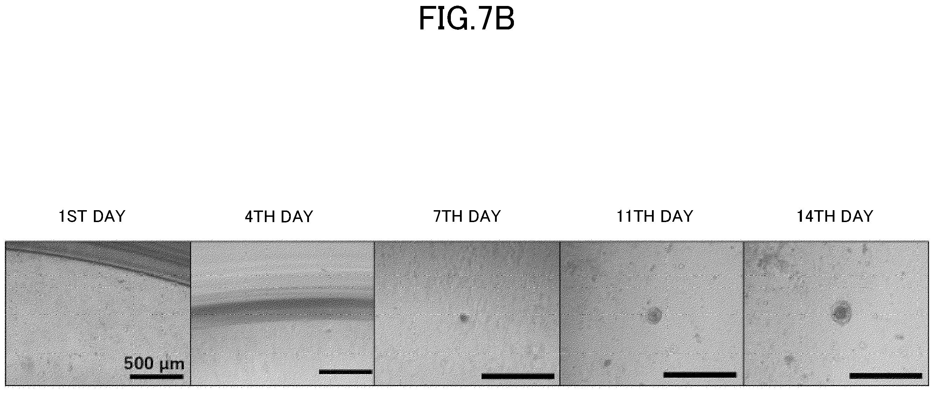

8.0×10⁴ hair follicle epithelial stem cells obtained in "1." were suspended in 20 µL of the epithelial cell culture medium prepared in (1), and 20 µL of Matrigel was added to prepare a total of 40 µL of a cell-gel mixed liquid. Then, the cell-gel mixed liquid was dropped to a 24-well plate (manufactured by BD Falcon) and incubated at 37°C for 30 minutes to harden Matrigel (see FIG. 4). Then, 500 µL of the epithelial cell culture medium was added, followed by culture for 14 days (hereinafter sometimes referred to as "related-art Matrigel-embedded culture method"). As a control, the hair follicle epithelial stem cells were inoculated into a 24-well plate (manufactured by BD Falcon) without being embedded in Matrigel, and were similarly cultured for 14 days (hereinafter sometimes referred to as "two-dimensional culture method"). The morphological changes of the cells over time (on the 1st, 4th, 7th, 11th, and 14th days of culture) were observed using an inverted phase contrast fluorescence microscope (manufactured by Olympus, IX-71). The results are shown in FIG. 7A (two-dimensional culture method) and FIG. 7B (related-art Matrigel-embedded culture method).

As shown in FIG. 7B, the hair follicle epithelial stem cells cultured by the related-art Matrigel-embedded culture method were dispersed from the 1st to 4th days of culture, but started to form a small aggregate on the 7th day of culture. The aggregate gradually increased in size, and reached a size of from about 100 µm to about 150 µm on the 14th day of culture.

Meanwhile, as shown in FIG. 7A and FIG. 7B, the hair follicle epithelial stem cells cultured by the two-dimensional culture method showed rapid cell growth compared to the hair follicle epithelial stem cells cultured by the related-art Matrigel-embedded culture method.

### (3) Analysis of Gene Expression Amount of CD34

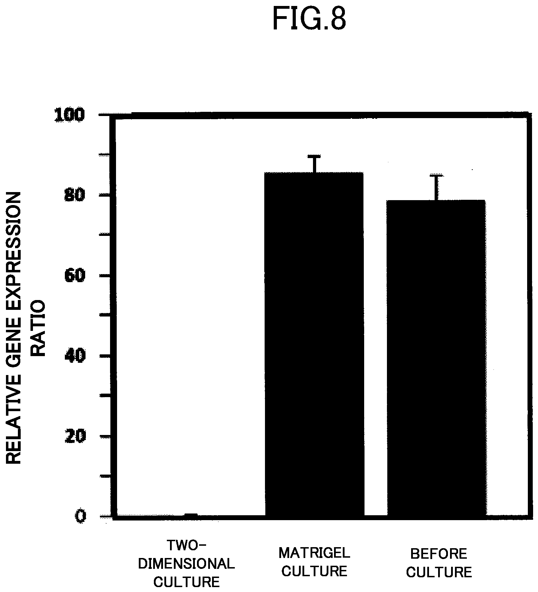

The gene expression amount of CD34 was analyzed using the same method as in "2. (5)" of Example 1 except that the hair follicle epithelial stem cells before culture, and the hair follicle epithelial stem cells on the 14th day of culture cultured by the related-art Matrigel-embedded culture method and the two-dimensional culture method, were used as samples. The results are shown in FIG. 6 and FIG. 8. In FIG. 6, Comparative Example 1 (low density) represents the gene expression amount of CD34 in the hair follicle epithelial stem cells on the 14th day of culture cultured by the Matrigel-embedded culture method. In addition, FIG. 8 shows the relative gene expression amounts of CD34 in the hair follicle epithelial stem cells on the 14th day of culture cultured by the Matrigel-embedded culture method and the hair follicle epithelial stem cells before culture when the gene expression amount of CD34 in the hair follicle epithelial stem cells on the 14th day of culture cultured by the two-dimensional culture method is defined as 1.

It was found from FIG. 8 that the hair follicle epithelial stem cells cultured by the related-art Matrigel-embedded culture method showed nearly the same gene expression amount of CD34 as the hair follicle epithelial stem cells before culture even on the 14th day of culture. Meanwhile, it was found that the hair follicle epithelial stem cells cultured by the two-dimensional culture method were considerably reduced in hair regeneration ability.

In general, it is known that when the contact of hair follicle epithelial stem cells with adjacent cells is broken owing to injury or the like, a strong growth switch is turned on, with the result that the hair follicle epithelial stem cells grow to cover a surface, to thereby prevent exogenous bacterial invasion. In this case, however, the cells, which have previously had a hair follicle-forming ability, are differentiated into cells for merely covering the surface. It is considered that, in the two-dimensional culture method, such an environment was unintentionally reproduced, and hence the hair regeneration ability was reduced, while in the related-art Matrigel-embedded culture method, the cells were able to be grown at a relatively mild rate, and hence the cells were able to be cultured while maintaining their hair regeneration ability.

In addition, it was found from FIG. 6 that, in the hair follicle epithelial stem cells cultured by the culture method according to this embodiment, the gene expression amount of CD34 was increased to be about 2.9 times as large as that in the hair follicle epithelial stem cells cultured by the related-art Matrigel-embedded culture method.

Thus, it was revealed that, in the case of culturing epithelial stem cells using Matrigel, it was more effective for maintaining their function to culture the cells using Matrigel after accumulating the cells in one place than to use Matrigel in a state in which the cells were uniformly dispersed.

### [Example 2] Culture of Hair Follicle Epithelial Stem Cells using Oxygen-permeable Cell Culture Vessel 1

### 1. Preparation of Hair Follicle Epithelial Stem Cells

Hair follicle epithelial stem cells were prepared using the same method as in "1." of Example 1.

### 2. Production of Oxygen-permeable Cell Culture Vessel

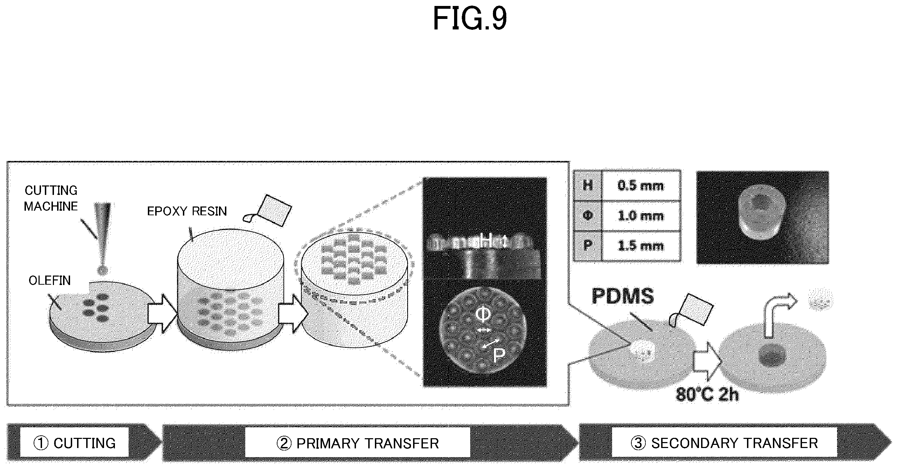

FIG. 9 is a schematic process diagram for illustrating a production method for an oxygen-permeable cell culture vessel (polydimethylsiloxane (PDMS) spheroid chip). The production method for an oxygen-permeable cell culture vessel is described in detail below with reference to FIG. 9.

First, through use of CAD software, V Carve Pro 6.5, the pattern of a spheroid vessel to be produced was designed on a computer. Then, through use of a cutting machine, an olefin-based substrate was cut according to the designed pattern to produce a concave mold having a pattern. An epoxy resin (CRYSTAL RESIN: manufactured by Nissin Resin Co., Ltd.) was poured into the concave mold, and cured for 1 day. Then, the concave mold was released to form a convex mold having a pattern. Then, the formed convex mold was fixed to the bottom surface of a 24-well plate (manufactured by BD Falcon), and polydimethylsiloxane (PDMS) was poured into the plate and solidified. Then, the convex mold was released. Thus, a PDMS spheroid chip in which a regular pattern was formed in PDMS was produced as an oxygen-permeable cell culture vessel. In the resultant PDMS spheroid chip, the depth of each well ("H" in FIG. 9) was 0.5 mm, the diameter of the opening portion of each well ("Φ" in FIG. 9) was 1.0 mm, the distance from the center of the opening portion of each well to the center of the opening portion of an adjacent well ("P" in FIG. 9) was 1.5 mm, and the radius of curvature (not shown) was 0.5 mm.

### 3. Culture of Hair Follicle Epithelial Stem Cells

### (1) Preparation of Epithelial Cell Culture Medium

An epithelial cell culture medium was prepared using the same method as in "2.(1)" of Example 1.

### (2) Culture of Hair Follicle Epithelial Stem Cells

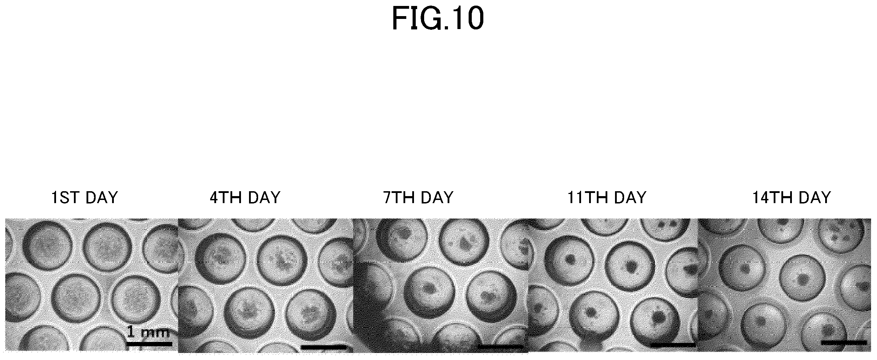

The hair follicle epithelial stem cells were cultured for 14 days using the same method as in "2. (2) to (4)" of Example 1 except that the oxygen-permeable cell culture vessel (PDMS spheroid chip) produced in "2." was used as the cell culture vessel. The morphological changes of the cells over time (on the 1st, 4th, 7th, 11th, and 14th days of culture) were observed using an inverted phase contrast fluorescence microscope (manufactured by Olympus, IX-71). The results are shown in FIG. 10.

As shown in FIG. 10, the hair follicle epithelial stem cells were deposited on the bottom surface of each well on the 1st day of culture, started to gradually aggregate on the 4th day of culture, formed one aggregate on the 7th day of culture, and maintained that shape to the 14th day of culture.

### (3) Analysis of Gene Expression Amount of CD34

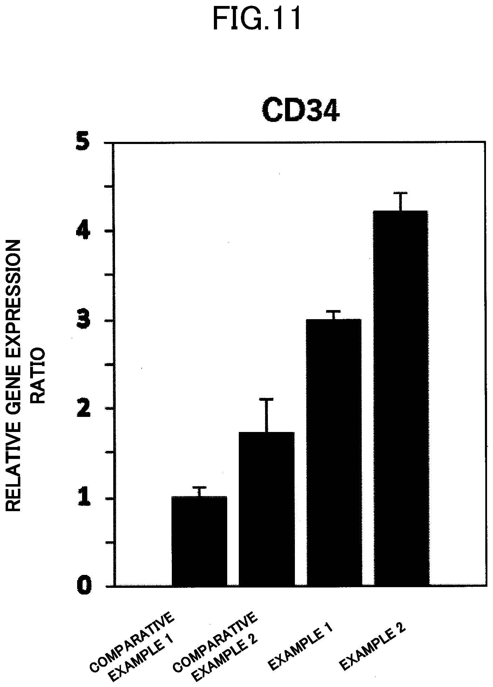

The gene expression amount of CD34 was analyzed using the same method as in "2. (5)" of Example 1 except that the hair follicle epithelial stem cells on the 14th day of culture cultured in (2) above were used as a sample. The results are shown in FIG. 11. FIG. 11 shows the relative gene expression amounts of CD34 in Example 1, Example 2, and Comparative Example 2 to be described below when the gene expression amount of CD34 in Comparative Example 1 (low density) described above is defined as 1. The discussion of the gene expression amount of CD34 in Example 2 shown in FIG. 11 will be described in Comparative Example 2 below.

### [Comparative Example 2] Culture of Hair Follicle Epithelial Stem Cells using Oxygen-permeable Cell Culture Vessel 2

### 1. Preparation of Hair Follicle Epithelial Stem Cells

Hair follicle epithelial stem cells were prepared using the same method as in "1." of Example 1.

### 2. Production of Oxygen-permeable Cell Culture Vessel

An oxygen-permeable cell culture vessel (PDMS spheroid chip) was produced using the same method as in "2." of Example 2.

### 3. Culture of Hair Follicle Epithelial Stem Cells (1) Preparation of Epithelial Cell Culture Medium

An epithelial cell culture medium was prepared using the same method as in "2.(1)" of Example 1.

### (2) Culture of Hair Follicle Epithelial Stem Cells

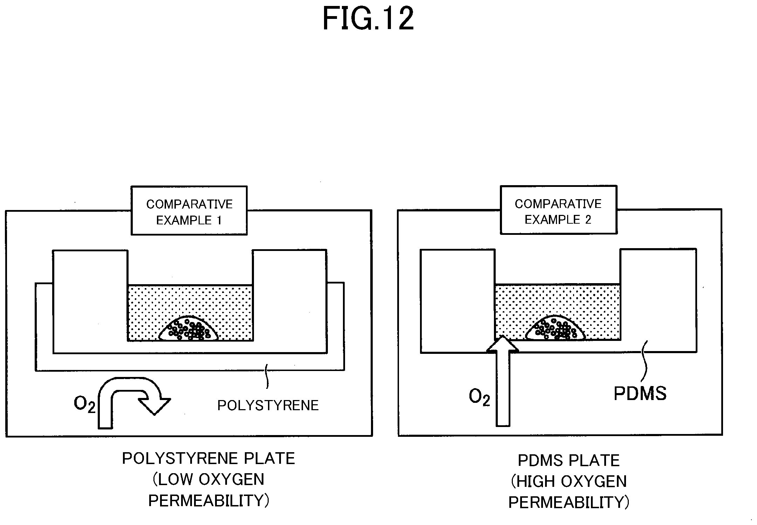

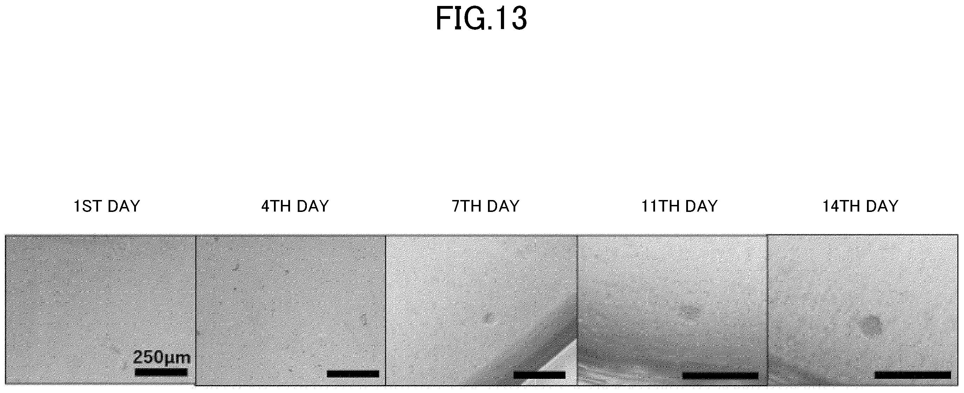

The hair follicle epithelial stem cells were cultured for 14 days using the same method as the related-art Matrigel-embedded culture method described in "2.(2)" of Comparative Example 1 except that the oxygen-permeable cell culture vessel (PDMS spheroid chip) produced in "2." was used as the cell culture vessel (see FIG. 12). The morphological changes of the cells over time (on the 1st, 4th, 7th, 11th, and 14th days of culture) were observed using an inverted phase contrast fluorescence microscope (manufactured by Olympus, IX-71). The results are shown in FIG. 13.

As shown in FIG. 13, the hair follicle epithelial stem cells were dispersed from the 1st to 4th days of culture, but formed a small aggregate on the 7th day of culture, and reached a size of from about 100 µm to about 150 µm on the 14th day of culture.

### (3) Analysis of Gene Expression Amount of CD34

The gene expression amount of CD34 was analyzed using the same method as in "2. (5)" of Example 1 except that the hair follicle epithelial stem cells on the 14th day of culture cultured in (2) above were used as a sample. The results are shown in FIG. 11. FIG. 11 shows the relative gene expression amounts of CD34 in Example 1, Example 2, and Comparative Example 2 when the gene expression amount of CD34 in Comparative Example 1 (low density) described above is defined as 1.

On the basis of the comparison of the micrographs of FIG. 7B and FIG. 13, no difference was found between the results of culture in the oxygen-permeable cell culture vessel (PDMS spheroid chip) and the results of culture in the 24-well plate.

However, on the basis of the comparison of the gene expression amounts of CD34 in Comparative Example 1 and Comparative Example 2 shown in FIG. 11, it was found that there was a significant difference in gene expression amount of CD34. Thus, it was found that a high-oxygen environment in which oxygen was more richly supplied was more effective for maintaining the function of hair follicle epithelial stem cells.

We take in oxygen through breathing, and the oxygen is transported through the body to cells by red blood cells and blood vessels running throughout the human body. The cells consume the oxygen to produce ATP from glucose through the citric acid cycle and the electron transport chain in mitochondria. The energy efficiency of this process is thermodynamically estimated to be about 43%. Meanwhile, under an anaerobic environment in which oxygen is absent, only the glycolytic system in the cells functions, and hence the energy efficiency is dramatically reduced and may be estimated to be about 2%. Therefore, when a low-oxygen state continues, the cells are expected to become unable to obtain energy for growing and maintaining their function.

Meanwhile, under a general culture environment, cells receive oxygen from a medium, and the medium is supplied with oxygen only from an air-liquid interface on a culture upper surface. Accordingly, a material balance cannot be maintained, and hence oxygen is consumed along with the progress of culture. The PDMS spheroid chip used in each of Example 2 and Comparative Example 2 is formed of an oxygen-permeable material, and hence the medium can be supplied with oxygen from all over the culture vessel. Therefore, it is conceivable that the culture of the hair follicle epithelial stem cells in the PDMS spheroid chip having high oxygen permeability increased the supply of oxygen to the culture medium to enable the cells to produce sufficient energy required for the growth of cells and the maintenance of their function.

In addition, as shown in FIG. 11, the hair follicle epithelial stem cells cultured by the culture method according to this embodiment using the PDMS spheroid chip (Example 2) showed a gene expression amount of CD34 about 4.2 times as large as that of the hair follicle epithelial stem cells cultured by the related-art Matrigel-embedded culture method (Comparative Example 1).

Thus, the potential of the PDMS spheroid chip to be useful for the culture of hair follicle epithelial stem cells was demonstrated.

### Industrial Applicability

According to the culture method and the culture kit according to the embodiments of the present invention, hair follicle epithelial stem cells are grown on a large scale while maintaining their hair regeneration ability. Hair follicle epithelial stem cells obtained by the culture method and the culture kit according to the embodiments of the present invention can be used to produce regenerated hair follicle primordia having an excellent hair regeneration ability.

## Claims

1. A culture method for hair follicle epithelial stem cells, comprising:
an accumulating step of forming an accumulation of hair follicle epithelial stem cells by inoculating a cell culture vessel with the hair follicle epithelial stem cells;
a mixing step of producing a mixture of an extracellular matrix component and the accumulation of the hair follicle epithelial stem cells by adding the extracellular matrix component to the accumulation of the hair follicle epithelial stem cells; and
a culturing step of culturing the hair follicle epithelial stem cells by adding a medium to the mixture.

2. The culture method for hair follicle epithelial stem cells according to claim 1, wherein the extracellular matrix component is type I collagen.

3. The culture method for hair follicle epithelial stem cells according to claim 1 or 2, wherein the cell culture vessel is formed of a material having oxygen permeability.

4. The culture method for hair follicle epithelial stem cells according to claim 3, wherein the material having oxygen permeability is polydimethylsiloxane.

5. A culture kit for hair follicle epithelial stem cells, comprising:
a cell culture vessel formed of a material having oxygen permeability;
an extracellular matrix component; and
a medium.


SEQUENCE LISTING

<110> National University Corporation YOKOHAMA National University
      Kanagawa Institute of Industrial Science and Technology

<120> METHOD FOR CULTURING HAIR FOLLICULAR EPITHELIAL STEM CELLS

<130> F1831PCT

<160> 4

<170> PatentIn version 3.5

<210> 1
<211> 21
<212> DNA
<213> Artificial Sequence

<220>
<223> Synthesized

<400> 1
tgggtcaagt tgtggtggga a                                                21

<210> 2
<211> 23
<212> DNA
<213> Artificial Sequence

<220>
<223> Synthesized

<400> 2
gaagaggcga gagaggagaa atg                                              23

<210> 3
<211> 20
<212> DNA
<213> Artificial Sequence

<220>
<223> Synthesized

<400> 3
agaacatcat ccctgcatcc                                                  20

<210> 4
<211> 20
<212> DNA
<213> Artificial Sequence

<220>
<223> Synthesized

<400> 4
tccaccaccc tgttgctgta                                                  20